Europäisches Patentamt

European Patent Office

Office européen des brevets

(11) Veröffentlichungsnummer: **0 212 060**
**A2**

(12) **EUROPÄISCHE PATENTANMELDUNG**

(21) Anmeldenummer: **86105147.2**

(22) Anmeldetag: **15.04.86**

(51) Int. Cl.⁴: **G01H 3/00**

(30) Priorität: **20.08.85 DE 3529704**

(43) Veröffentlichungstag der Anmeldung:
**04.03.87 Patentblatt 87/10**

(84) Benannte Vertragsstaaten:
**CH DE GB IT LI**

(71) Anmelder: **ROBERT BOSCH GMBH**
**Postfach 50**
**D-7000 Stuttgart 1(DE)**

(72) Erfinder: **Köpke, Wolfgang, Dipl.-Ing.**
**Prinzregentenstrasse 7**
**D-1000 Berlin 31(DE)**

(74) Vertreter: **Schmidt, Hans-Ekhardt**
**Robert Bosch GmbH Geschäftsbereich**
**Elektronik Patent- und Lizenzabteilung**
**Forckenbeckstrasse 9-13**
**D-1000 Berlin 33(DE)**

(54) **Verfahren zum Kalibrieren elektroakustischer Messgeräte.**

(57) Es wird ein Verfahren vorgeschlagen, das zum Kalibrieren elektroakustischer Meßgeräte, insbesondere Audiometer, vorgesehen ist. Das Verfahren arbeitet automatisch, wobei die Kalibrierwerte des Meßgrätes (10) als Digitalwerte in einem nichtflüchtigen elektrisch veränderbaren elektronischen Speicher (15) abgelegt werden. Aus diesem Speicher werden sie im Meßbetrieb des Meßgerätes zwecks Korrektur der Meßwerte abgerufen. Mit dem Meßgerät ist ein elektronischer Rechner (22) verbunden. Der Rechner vergleicht die von dem Meßgerät gelieferten Istwerte mit in dem Rechner gespeicherten Sollwerten und schreibt diese in den elektronischen Speicher (15) ein.

Fig. 1

EP 0 212 060 A2

## Verfahren zum Kalibrieren elektroakustischer Meßgeräte

### Stand der Technik

Die Erfindung geht von einem Verfahren nach der Gattung des Anspruchs 1 aus.

Es ist bekannt (Fiedler/Wassenberg, Handbuch der Medizintechnik, 2. Ergänzungslieferung, Februar 1984, II-2.1.11 Anhang, Seiten 5 bis 7), Audiometer manuell zu kalibrieren. Der damit verbundene Aufwand ist jedoch sehr zeit-und kostenintensiv. Weiterhin ist es bekannt, das Kalibrieren von elektroakustischen Meßgeräten zu automatisieren. Die bisher bekanntgewordenen automatischen Kalibrierverfahren haben jedoch den Nachteil, daß die ermittelten Kalibrierwerte bei einem Ausfall der Betriebsspannung oder nach dem Abschalten derselben verlorengehen.

### Vorteile der Erfindung

Das erfindungsgemäße Verfahren mit den kennzeichnenden Merkmalen des Anspruchs 1 hat den Vorteil, daß bei einem automatischen Kalibrierverfahren die ermittelten Kalibrierwerte auch nach dem Ausfall oder Ausschalten der Betriebsspannung erhalten bleiben.

Durch die in den Unteransprüchen aufgeführten Maßnahmen sind vorteilhafte Weiterbildungen und Verbesserungen des im Anspruch 1 angegebenen Verfahrens möglich. Besonders vorteilhaft ist ein erfindungsgemäßes Verfahren, bei dem das Meßgerät mit einem elektronischen Rechner verbunden wird, der die Istwerte des Meßgerätes mit den in dem Rechner gespeicherten Sollwerten vergleicht und die durch den Vergleich erhaltenen Kalibrierwerte in den elektronischen Speicher einschreibt bzw. in diesem überschreibt. Auf diese Weise enthält der elektronische Speicher jederzeit die neuesten Kalibrierwerte.

### Zeichnung

Ein Ausführungsbeispiel der Erfindung ist in der Zeichnung an Hand zweier Figuren dargestellt und in der nachfolgenden Beschreibung näher erläutert. Die Zeichnung zeigt in

Fig. 1 ein Blockschaltbild einer Vorrichtung zur Durchführung des erfindungsgemäßen Kalibrierverfahrens und

Fig. 2 ein Blockschaltbild einer Kalibirierspeichervorrichtung, einer Kalibrierschaltvorrichtung und einer Kalibrierteilervorrichtung.

### Beschreibung der Erfindung

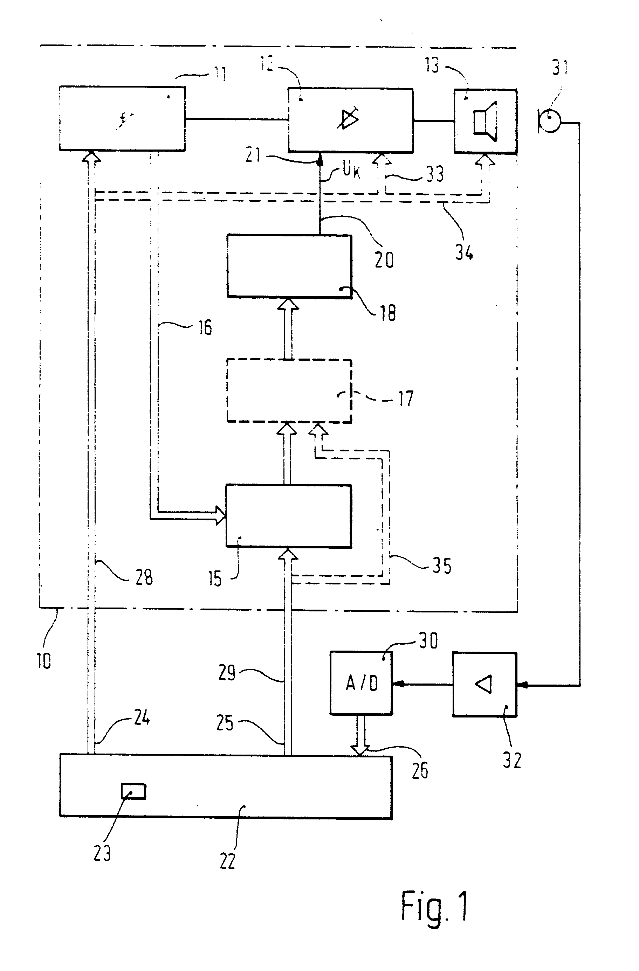

In dem Blockschaltbild nach Fig. 1 bedeutet der durch strichpunktierte Linien umrahmte Schaltungsteil ein Audiometer 10. Dieses enthält einen Tongenerator 11, einen Verstärker 12 und einen Schallwandler 13, das ist vorzugsweise ein Lautsprecher. Die Frequenz des Tongenerators 11 und die Verstärkung des Verstärkers 12 können in Stufen eingestellt werden. Das Audiometer weist über die bekannten Baueinheiten hinaus einen nichtflüchtigen elektrisch veränderbaren elektronischen Speicher 15 auf, das ist vorzugsweise ein EEPROM-Baustein, der von dem Tongenerator 11 über einen Adreßbus 16 angesteuert wird. An den elektronischen Speicher 15 schließt sich gegebenenfalls über eine Kalibrierschaltvorrichtung 17, die in Fig. 1 durch gestrichelte Linien gekennzeichnet ist, eine Kalibrierteilervorrichtung 18 an. Ein Ausgang der Kalibrierteilervorrichtung steht über eine Leitung 20 mit einem Steuereingang 21 des Verstärkers 12 in Verbindung. Dem vorstehend beschriebenen Audiometer 10 ist ein elektronischer Rechner 22 zugeordnet, der eine Kalibriertaste 23, einen ersten Ausgang 24, einen zweiten Ausgang 25 und einen Eingang 26 aufweist. Der erste Ausgang 24 steht über einen Adreßbus 28 mit dem Tongenerator 11, der zweite Ausgang 25 über einen Daten-und Programmbus 29 mit dem elektronischen Speicher 15 und der Eingang 26 mit einem Digitalausgang eines Analog/Digital-Wandlers 30 in Verbindung. Mit dem Schallwandler 13 des Audiometers 10 ist ein Meßmikrofon 31 gekuppelt, das über einen Verstärker 32 mit dem Analogeingang des Analog/Digital-Wandlers 30 in Verbindung steht.

Die vorstehend beschriebene Kalibriervorrichtung für ein Audiometer 10 hat folgende Funktion.

Liegt die Kalibriervorrichtung an der Betriebsspannung und wird die Kalibriertaste 23 des elektronischen Rechners 22 betätigt, so läuft das in dem Rechner gespeicherte Kalibrierprogramm automatisch ab. Zunächst schaltet der Rechner 22 über den Adreßbus 28 den Tongenerator 11 auf eine erste Tonfrequenz $f_1$. Das von dem Tongenerator abgegebene Tonfrequenzsignal wird in dem Verstärker 12 verstärkt, wobei ein Pegelsteller des Verstärkers von Hand in die Kalibrierposition übergeführt wird. Der Tongenerator 11 liefert außerdem über den Adreßbus 16 ein der Tonfre-

quenz $f_1$ entsprechendes Adreßsignal (= Datenwort) an den Speicher 15. Dieser gibt an die Kalibrierschaltvorrichtung 17 ein dem Adreßsignal entsprechendes, in dem Speicher 15 gespeichertes Datenwort ab, wodurch die Kalibrierteilervorrichtung 18 derart umgeschaltet wird, daß sie an ihrem Ausgang eine bestimmte Kalibrierspannung $U_K$ abgibt. Diese Spannung bewirkt eine Korrektur des von Hand eingestellten Kalibrierpegels. Alternativ kann der Pegelsteller auch über einen von dem Adreßbus 28 abzweigenden weiteren Adreßbus 33 durch den Rechner 22 automatisch in die erforderliche Kalibrierposition geschaltet werden.

Das mit dem Verstärker 12 verstärkte Tonsignal der Frequenz $f_1$ wird über den Schallwandler 13 des Audiometers 10 wiedergegeben und von dem Meßmikrofon 31 aufgenommen. Die von dem Meßmikrofon abgegebene Meßspannung wird nach Verstärkung mit dem Verstärker 32 mit dem Analog/Digital-Wandler 30 in einen entsprechenden Digitalwert umgewandelt. Das dem Istpegel der Mikrofonspannung entsprechende digitale Datenwort wird dem Eingang 26 des Rechners 22 zugeführt. In dem Rechner wird der Istpegelwert mit einem in dem Rechner gespeicherten Sollpegelwert verglichen. Besteht zwischen Istwert und Sollwert keine Abweichung, so schaltet der Rechner 22 über den Adreßbus 28 den Tongenerator 11 auf die nächste Tonfrequenz, zum Beispiel $f_2$, um, und der Kalibriervorgang läuft analog wie bei der Tonfrequenz $f_1$ ab. Stellt der Rechner 22 dagegen eine Differenz zwischen dem Ist-und dem Sollwert fest, so wird ein dem Differenzwert entsprechendes Datenwort über den Daten-und Programmierbus 29 in den Speicher 15 eingeschrieben bzw. der darin gespeicherte Wert überschrieben. Über die Kalibrierschaltvorrichtung 17 und die Kalibrierteilervorrichtung 18 wird dann ein neuer Kalibrierspannungswert $U_K$ an den Steuereingang 21 des Verstärkers 12 abgegeben.

Dieser Kalibriervorgang wird, durch den Rechner 22 gesteuert, noch einmal wiederholt, das heißt der von dem Meßmikrofon 31 gelieferte Istpegelwert wird in digitaler Form in dem Rechner mit dem Sollwert verglichen, und falls noch eine Differenz zwischen Soll-und Istwert besteht, eine entsprechende Nachsteuerung der Verstärkung vorgenommen. Erst wenn keine Differenz zwischen Soll-und Istwert mehr besteht, erfolgt die Umschaltung des Tongenerators 11 auf die nächste Tonfrequenz $f_3$.

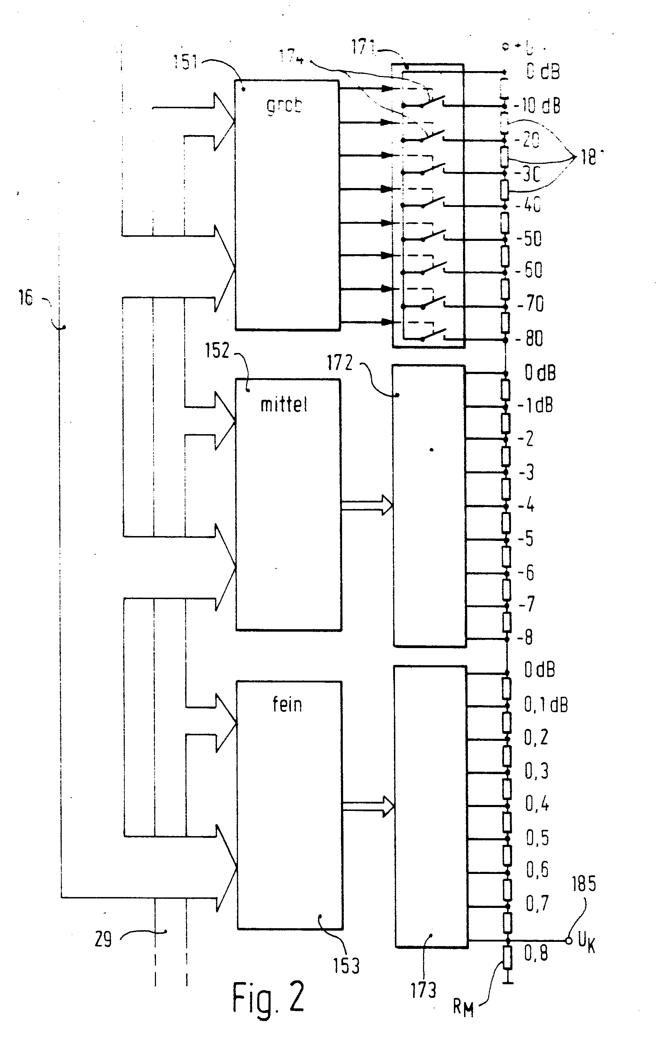

In Fig. 2 ist ein Schaltbild einer Grob-, Mittel-, Fein-Kalibriersteuerung gezeigt. Der elektronische Speicher 15 gemäß Fig. 1 ist bei dem Ausführungsbeispiel in Fig. 2 in drei Kalibrierspeicher 151 ("grob"), 152 ("mittel") und 153 ("fein") aufgeteilt. Die Kalibrierspeicher werden durch ein der eingestellten Tonfrequenz entsprechendes Datenwort des Tongenerators 11 über den Adreßbus 16 (vgl. Fig. 1) parallel angesteuert. Die in den Speicherzellen der Kalibrierspeicher gespeicherten und durch das Datenwort abgerufenen digitalen Informationen steuern drei den Kalibrierspeichern zugeordnete Schaltvorrichtungen 171, 172 und 173. Die Schaltvorrichtung 171 enthält ebenso wie die anderen Schaltvorrichtungen 172 und 173 acht Schalter 174, mit denen aus einer Kette von Widerständen 181, die der Kalibrierteilervorrichtung 18 in Fig. 1 entsprechen, Widerstände kurzgeschlossen werden können. Die Kette von Widerständen 181 liegt zwischen einer Spannung $+U$ und Masse. Die Kalibrierspannung $U_K$ wird zwischen einem Anschluß 185 und Masse, das heißt an einem Meßwiderstand $R_M$ abgegriffen. Je nach den Widerstandswerten der nichtüberbrückten Widerstände stellt sich eine bestimmte Kalibrierspannung $U_K$ ein. Die Widerstandswerte sind so gewählt, daß sich die in Fig. 2 neben den Widerständen gezeigten Abstufungen 0 ... 80 db, 0 ... 8 db und 0 ... 0,8 dB ergeben.

Altvernativ zu den in den Fig. 1 und 2 gezeigten Ausführungsbeispielen kann das Kalibrierverfahren mit folgenden zusätzlichen Merkmalen versehen sein.

Über einen Adreßbus 34 des Rechners 22 (Fig. 1) können vorzugsweise auch andere Schallwandler, wie zum Beispiel ein Knochenleitungshörer oder ein Luftleitungshörer, eingeschaltet werden, wobei deren von dem Lautsprecher abweichende Charakteristik in dem Rechner 22 abgelegt ist.

Über den Adreßbus 28 kann der Rechner 22 anstelle des in Fig. 1 gezeigten Tongenerators 11, der normalerweise nur sinusförmige Tonsignale liefert, zum Beispiel einen Rauschgenerator einschalten.

Eine weitere Variante besteht darin, daß der Rechner 22 über den Adreßbus 28 den Tongenerator 11 veranlaßt, eine amplitudenmodulierte Wechselspannung abzugeben, die zum Beispiel für den sogenannten SISI-Test benötigt wird.

Schließlich kann es zur beschleunigten Sollwerteinstellung beim Kalibrieren nützlich sein, den Daten-und Programmbus 29 des Rechners 22 unter Umgehung des elektronischen Speichers 15 - (vgl. gestrichelt eingezeichneter Bus 35) direkt mit der Kalibrierschaltvorrichtung 17 zu verbinden. Erst wenn der Istwert dem Sollwert angeglichen ist, wird er neue Kalibrierwert in dem Speicher 15 eingeschrieben bzw. überschrieben.

## Ansprüche

1. Verfahren zum Kalibrieren elektroakustischer Meßgeräte, vorzugsweise Audiometer, dadurch gekennzeichnet, daß die Kalibrierwerte des Meßgerätes (10) als Digitalwerte in einem nichtflüchtigen elektrisch veränderbaren elektronischen Speicher (15) abgelegt werden, aus dem sie im Meßbetrieb des Meßgerätes zwecks Korrektur der Meßwerte automatisch abgerufen werden.

2. Verfahren nach Anspruch 1, dadurch gekennzeichnet, daß das Meßgerät (10) mit einem elektronischen Rechner (22) verbunden wird, der die Istwerte des Meßgerätes mit den in dem Rechner gespeicherten Sollwerten vergleicht und die durch den Vergleich erhaltenen Kalibrierwerte in den elektronischen Speicher (15) einschreibt bzw. in diesem überschreibt.

3. Verfahren nach Anspruch 2, dadurch gekennzeichnet, daß die in dem elektronischen Speicher (15) abgelegten Kalibrierwerte gegebenenfalls über eine Kalibrierschaltvorrichtung (17) eine mit dieser verbundene Kalibrierteilervorrichtung (18) steuern.

4. Vorrichtung zur Durchführung des Verfahrens nach Anspruch 1, 2 oder 3, dadurch gekennzeichnet, daß der elektronische Speicher (15) die Kalibrierschaltvorrichtung (17) und die Kalibrierteilervorrichtung (18) in Stufen (grob, mittel, fein) unterteilt sind.

5. Vorrichtung zur Durchführung des Verfahrens nach Anspruch 1, 2 oder 3, dadurch gekennzeichnet, daß der elektronische Speicher (15) ein EEPROM-Speicher ist.

6. Vorrichtung zur Durchführung des Verfahrens nach einem der Ansprüche 1 bis 3, dadurch gekennzeichnet, daß bei einem Audiometer (10) mit einem Tongenerator (11), einem Verstärker (12) und einem Schallwandler (13) mit dem Schallwandler ein Meßmikrofon (31) akustisch gekoppelt ist und daß das Meßmikrofon über einen Verstärker (32) und einen Analog/Digital-Wandler (30) mit dem elektronischen Rechner (22) verbunden ist.

7. Vorrichtung nach Anspruch 6, dadurch gekennzeichnet, daß der Schallwandler (13) über einen Adreßbus (34) des elektronischen Rechners (22) auf verschiedene Schallwandlertypen umschaltbar ist, deren Charakteristik in dem Rechner gepeichert ist.

8. Vorrichtung nach einem der Ansprüche 4 bis 7, dadurch gekennzeichnet, daß der Tongenerator (11) über den Adreßbus (28) des Rechners (22) auf verschiedene Betriebsarten, wie zum Beispiel Sinussignalgenerator oder Rauschsignalgenerator, umschaltbar ist.

9. Vorrichtung nach Anspruch 8, dadurch gekennzeichnet, daß der Tongenerator (11) über den Adreßbus (28) auf die Erzeugung einer amplitudenmodulierten Wechselspannung umschaltbar ist.

10. Vorrichtung nach Anspruch 4, dadurch gekennzeichnet, daß die Kalibrierteilervorrichtung (18) aus einer Kette von Widerständen (181) besteht, die zwischen einer positiven Spannung (U) und einem festen Bezugspotential liegt, daß die einzelnen Widerstände durch Kalibrierschaltvorrichtungen (171) in beliebiger Kombination überbrückbar sind und daß die Kalibrierspannung ($U_K$) an einem Meßwiderstand ($R_M$) der Kette von Widerständen abgreifbar ist.

Fig. 1

0 212 060

37/85

0 212 060

Fig. 2